# EUROPEAN PATENT SPECIFICATION

(11) **EP 2 983 630 B1**
(45) Date of publication and mention of the grant of the patent: **24.06.2020**
(21) Application number: 14837724.5
(22) Date of filing: 27.05.2014
(51) Int. Cl.: A61F 9/007, A61B 3/103, A61B 3/16

(54) **SYSTEMS FOR INTRA-OPERATIVE EYE BIOMETRY OR REFRACTIVE MEASUREMENT**
SYSTEME ZUR INTRAOPERATIVEN AUGENBIOMETRIE ODER REFRAKTIVEN MESSUNG
SYSTÈMES POUR MESURE DE BIOMÉTRIE OU RÉFRACTIVE DE L' OEIL PEROPÉRATOIRE

(30) Priority: 22.08.2013 US 201313972975
(43) Date of publication of application: 17.02.2016
(73) Proprietor: Alcon Inc., 1701 Fribourg (CH)
(72) Inventor: ARTSYUKHOVICH, Alexander N., Irvine, California 92619 (US); ASLAN, Aras, Foothill Ranch, California 92610 (US); YU, Lingfeng, Rancho Santa Margarita, California 92688 (US)
(74) Representative: Hanna Moore + Curley
(86) International application number: PCT/US2014/039579
(87) International publication number: WO 2015/026414

(56) References cited:
- WO-A2-2012/137067
- US-A1- 2008 086 048
- US-A1- 2012 215 160
- US-A1- 2012 238 857
- US-A1- 2012 296 423
- US-A1- 2013 085 370
- US-A1- 2013 158 381
- US-B1- 6 443 893

## Description

### BACKGROUND

The present disclosure relates to systems for intra-operative eye refractive measurement, and more particularly, to systems using a smart intraocular pressure valve and tonometer for intra-operative eye refractive measurement.

### BACKGROUND

Typical phacoemulsification surgical procedures include pre-surgical optical analysis of the eye. The pre-surgical analysis includes measurement of tissue and anatomical features when the eye is whole and eye pressure is at a natural or normal level. Based on the pre-surgical analysis, a surgical plan is created. The surgical plan takes into account the tissue and the size of different anatomical features of the eye. In addition, based on this, the refractive power of a replacement lens is also selected. However, during the surgical procedure, where tissue is removed and manipulated, the size of the different anatomical features may change, which may affect the fit of the earlier-selected replacement lens. Current state-of-the art cataract surgery relies on semi-empirical IOL formulae to prescribe refractive power of an IOL based on a patient's pre-op biometry data. The surgeon is often left with the difficult task of prescribing a single IOL refractive power based on differing IOL formulae answers. For the best surgical outcome, the surgeon must often make choices based on past experience, interpretation of various biometry measurements, data clustering, and trending for example.

In addition, since the surgical procedure typically occurs when the eye is at an elevated intra ocular pressure or IOP, any intraoperative measurements without normalizing IOP do not correspond to the size of the anatomical features as they will be when the eye is in its normal or natural state.

The present disclosure addresses one or more deficiencies in the prior art.

Reference is made to US2008/00868048 which relate to a method for measuring biomechanical properties in an eye. A perturbation component introduces a stress to the eye tissue. An imaging component is operative to obtain an image of the eye tissue. A first image of the tissue can be obtained prior to the introduction of the stress and a second image of the tissue can be obtained after the introduction of the stress. An image analysis component compares the first image and the second image as to determine at least one biomechanical property of the tissue. It is described that pressure is measured in a chamber coupled to an eye.

### SUMMARY

In an exemplary aspect, the present disclosure is directed to a system for capturing intraoperative biometry and/or refractive measurements. The system includes a sensor associated with the eye and configured to detect a pressure of the eye. The system also includes an intra-op diagnostics device including a control unit arranged to actuate the intra-op diagnostics device to capture the intraoperative biometry and/or refractive measurements when the sensor detects pressure within a pressure range.

In an aspect, the system also includes a plug, with the sensor being carried on the plug. The plug may be sized for implantation within the eye of the patient during the surgical procedure. In an aspect, the plug comprises a first transmission module in communication with the sensor, and the intra-op diagnostics device comprises a second transmission module in communication with the control unit. The first transmission module may be in communication with the second transmission module. The control unit may capture the intraoperative biometry and/or refractive measurements when the communication from the first transmission module indicates that the pressure is within the pressure range.

In an aspect, the plug comprises a valve configured to open and close to increase and decrease drainage from the eye to affect the pressure in the eye. In an aspect, the valve is configured to close when the pressure in the eye reaches the pressure threshold. In an aspect, the pressure threshold is defined in terms of IOP.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description. The invention is limited to the subject-matter covered by the claims. Any mentioned or described surgical or therapeutic methods are not intended to be part of the invention, but only presented for illustrative purposes.

### BRIEF DESCRIPTION OF THE DRAWINGS

The accompanying drawings illustrate embodiments of the devices herein and together with the description, serve to explain the principles of the present disclosure.
Fig. 1 is an illustrative schematic of an exemplary intraoperative measurement system in accordance with an aspect of the present disclosure.
Fig. 2 is a block diagram of a plug of the exemplary intraoperative measurement system in accordance with an aspect of the present disclosure.
Fig. 3 is a graph showing IOP of an eye over time in accordance with an aspect of the present disclosure.
Fig. 4 is a block diagram of a plug of the exemplary intraoperative measurement system in accordance with an aspect of the present disclosure.
Fig. 5 is a graph showing IOP of an eye over time in accordance with an aspect of the present disclosure.
Fig. 6 is a block diagram of a system of an exemplary intraoperative measurement system in accordance with an aspect of the present disclosure.
Fig. 7 is a block diagram of a system of an exemplary intraoperative measurement system in accordance with an aspect of the present disclosure.
Fig. 8 is a flow chart showing an exemplary method of performing a surgical procedure, which is not part of the present invention.
Fig. 9 is a flow chart showing an exemplary method of performing a surgical procedure, the method not being part of the invention.

### DETAILED DESCRIPTION OF THE PREFERRED EMBODIMENTS

For the purposes of promoting an understanding of the principles of the present disclosure, reference will now be made to the exemplary embodiments illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. For simplicity, in some instances the same reference numbers are used throughout the drawings to refer to the same or like parts.

The present disclosure relates generally to systems for intraoperative biometry and/or refractive measurement in cataract surgery and phakic IOL implantation. Intra-op diagnostics may be used to perform actual refractive measurements on an aphakic eye. Such diagnostics were previously not possible to perform due to refractive distortions, introduced by cataract. Here however, intra-op diagnostics rely on direct refractive measurements rather than biometric measurements and complex IOL formulae that predict refractive error. During typical ocular surgical procedures, the eye is maintained at an elevated pressure or IOP in order to ensure that there is no collapse at the eye. Accordingly, during the procedure, or shortly thereafter, the pressure or IOP is permitted to decrease to a more natural or stabilized level. In some aspects, this may be around 30mmHg or lower. Some systems described herein include using a valve opened to allow pressure or IOP to gradually decrease. The valve may close when the pressure or IOP reaches a natural or desired level. At this time, the surgeon may capture biometry and/or refractive measurements using an intra-op diagnostics device. Since the measurements are taken when the pressure or IOP is close to the natural or desired level, the measurements reflect the size of the eye when it is in its natural condition, instead of its inflated or stretched condition. As such, when a lens is later selected and implanted, it fits properly when the eye is in its natural state having a natural pressure or IOP.

Other embodiments described herein include valves or sensors that control pressure or IOP to match the eye's natural or desired state while capturing biometry and/or refractive measurements. The system may capture biometry and/or refractive measurements at a particular instant in time as the pressure or IOP reaches the natural or desired pressure or IOP or may capture it over a period of time. Some embodiments capture pressure or IOP measurements automatically, thus streamlining procedures and improving precision of adjustments.

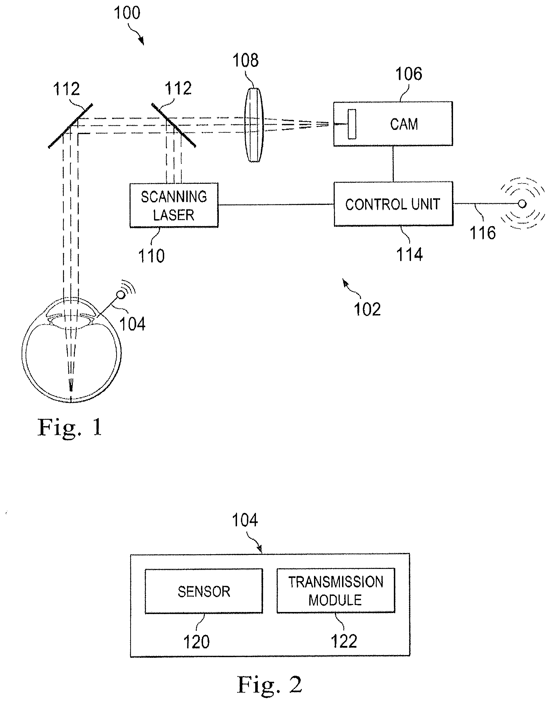

Fig. 1 shows an exemplary stylized embodiment of a system 100 for taking intraoperative biometry and/or refractive measurements in cataract surgery and phakic IOL implantation. The system 100 includes an intra-op diagnostics device 102 and a plug 104. The intra-op diagnostics device 102 may include, for example, an aberrometer and an optical coherence tomographer (OCT), which capture biometry and/or refractive measurements of an eye, while the plug 104 operates based on eye pressure. The intra-op diagnostics device may also include other eye measurements instruments, like wavefront sensors, video cameras and such. The plug 104 may also include a valve (described below) that closes to reduce drainage when the pressure or IOP level reaches the desired level, which may be selected to correspond to the natural pressure or IOP level for the individual patient. The natural IOP level may be a level measured pre-surgery, an average level taken over a period of time, or other level intended to relate the natural state of the IOP level.

The intra-op diagnostics device 102 includes a camera 106, a lens 108, a scanning laser generator 110, reflective units 112, and a control unit 114. The camera 106, the lens 108, the scanning laser generator 110, and the reflective units 112 are conventional and are not described further. The OCT device forming a part of the intra-op diagnostics device 102 is conventional and is not described further. Depending upon the embodiment, the control unit 114 may be specifically configured to cooperate with the plug 104 to control the pressure or IOP within the eye. The control unit 114 typically includes a processor and memory, with the processor being, for example only, an integrated circuit with power, input, and output pins capable of performing logic functions, or a controller that controls different components that perform different functions. To perform OCT functions, the control unit 114 also may include an optical fiber interferometer and high-speed analog to digital converter. The memory may be a semiconductor memory that interfaces with the processor. In one example, the processor can write to and read from the memory. For example, the processor can be configured to read data from the plug 104 and write that data to the memory. In this manner, a series of sensed or calculated pressure or IOP readings can be stored in the memory. The processor is also capable of performing other basic memory functions, such as erasing or overwriting the memory, detecting when the memory is full, and other common functions associated with managing semiconductor memory.

Some embodiments of the control unit 114 include a data transmission module 116 employing any of a number of different types of data transmission. For example, data transmission module 116 may be an active device such as a radio. Data transmission module 116 may also be a passive device such as an antenna and receiver.

The data transmission module 116 may communicate with the plug 104 and may pass signals representing information received from the plug 104 to the control unit 114 for processing. In some embodiments, the transmission module 116 comprises a receiver that receives wireless signals form the plug 104 representing, for example, information relating to the state of the plug 104 or pressure data relating to the actual measured pressure or IOP of the eye. In other embodiments, the transmission module 116 includes both a receiver and a transmitter for communicating with the plug 104. Depending on the embodiment, the data transmission module 116 and the plug 104 are configured for wired communication or wireless communication, and may communicate over Wi-Fi, Bluetooth, wireless local area network (WLAN), or wireless personal area networks (WPAN).

The plug 104 is configured to be associated with the eye and may be used to detect pressures or control the rate of drainage from the eye intraoperatively or at the conclusion of a surgical procedure. Detected pressures may be communicated to the control unit 114. Drainage control may be performed based upon signals from the control unit 114.

Typically, phacoemulsification surgery results in two small incisions being made into the eye. The larger of these receives the phacoemulsification handpiece tip to perform the phacoemulsification procedure. The smaller of these receives an assistance instrument, for example, a pick to move cataract pieces toward phaco tip, for example. As indicated previously, pressure or IOP is typically elevated during the surgical process in order to reduce a chance that the eye may collapse during the procedure. Therefore, when the surgical process ceases, the drainage from one or both of the incisions gradually decreases the pressure or IOP from its elevated surgical state toward the more natural normal state. With the incisions open, the pressure will fall below the natural or normal pressure or IOP level. Self-sealing structured incisions may reduce this occurrence, but some leakage may still occur at elevated IOP.

The plug 104 is sized and arranged to fit within one of the incisions and may operate as a restriction in the incision that reduces or inhibits drainage. Accordingly, intraoperatively, the phacoemulsification handpiece or the irrigation tube may be withdrawn, and the plug 104 may be inserted into the incision so that intraoperative biometry and/or refractive measurements may be obtained.

The plug 104 may comprise a pressure sensor and/or a flow control element, such as a valve. In embodiments employing a valve, the valve may comprise a passive valve, a pressure driven valve, an electronically controlled valve or other type of valve and may affect flow of fluid from the eye after a portion of the surgical procedure. It may include any number of valves and valve types in combination. Some embodiments also include one or more pumping systems that cooperate with one or more valves to maintain pressure or IOP stability. As will be described below, the valve 104 may operate under the control of the control unit 114 and may receive instructions from the control unit 114 to permit increased flow of fluid from the anterior chamber or to decrease flow to meet a desired flow rate and pressure.

Fig. 2 shows an embodiment of the plug 104 in block diagram form. In Fig. 2, the plug 104 includes a sensor 120 and a transmission module 122. The sensor 120 can be any type of pressure sensor suitable for detecting the pressure in the eye. Further, the sensor 120 may comprise more than one pressure sensor. The transmission module 122 is configured to transmit signals to the control unit 114 that represents the pressure measured by the sensor 120. The transmission module 122 may be similar to the transmission module 116 and the description above also applies to the transmission module 122.

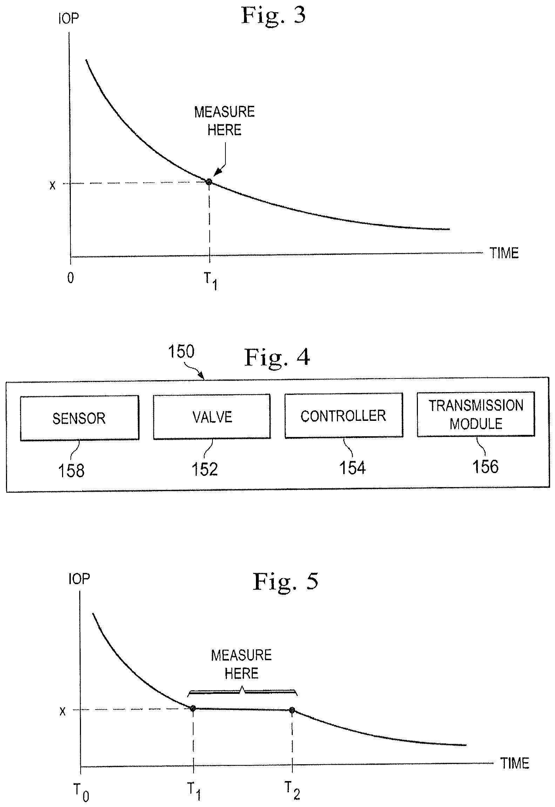

Fig. 3 is a graph showing IOP measured over a time period intraoperative or immediately following a phacoemulsification procedure, and identifying a point in time when the IOP matches a target IOP selected to correspond to a natural IOP for a patient. At the time T0 along the horizontal axis, the surgeon may pause the surgical procedure or withdraw the irrigation tube from the eye or turn off irrigation flow to the eye. Accordingly, at time T0, the eye is inflated due to the higher IOP maintained during surgery. Also at time T0, the plug 104 may be inserted into one of the incisions or access ports into the eye. The plug 104, therefore, measures the pressure in the eye via the sensor 120, and the pressure is transmitted by the transmission module 122 to the control unit 114. As irrigation fluid drains from the eye through the incisions, the eye pressure and the corresponding IOP decreases over time along the exponential curve shown in Fig. 3. The control unit 114 controls the intra-op diagnostics device 102 to take biometry and/or refractive measurements when the IOP reaches a target pressure or passes through a target range surrounding a target pressure. In Fig. 3, the target pressure is identified by IOP X along the IOP axis. Accordingly, intraoperative biometry and/or refractive measurements may be obtained when the IOP is at a normal state, rather than an elevated state. This allows the surgeon to select a lens for implantation that may have a better fit than when the lens is selected based on pre-surgery biometry and/or refractive measurements and a better fit than when the lens is selected based on measurements taken with the eye at an elevated or non-normal IOP. While shown with IOP, the same principles apply if the system relies upon straight measured pressure.

Fig. 4 shows another plug 150 that may be used with the intra-op diagnostics device 102 in place of the plug 104 described above. In this embodiment, the plug 150 includes a valve 152, an optional controller 154, an optional transmission module 156, and an optional sensor 158. In some embodiments, the valve 152 is a passive valve, such as a mechanically operated valve arranged to open when pressure on one side of the valve exceeds an established threshold, and is configured to close when the pressure meets or drops below the threshold. The threshold may be a specific value or a range of values. Some of these types of valves may include a controlled outflow orifice. For example, the valve 152 may be a ball valve configured to open when the pressure in the eye is greater than a certain value and close when the valve is below the certain value. Accordingly, the valve 152 may be open during the period of higher pressure or IOP and may be closed when the pressure or IOP is close to or at normal pressure or IOP. This may be done to lengthen the period of time when the pressure or IOP is at a normal state during the drainage process in the eye. Lengthening the time that the pressure or IOP is at a normal state provides additional time to take measurements with the intra-op diagnostics device. Other types of mechanical type valves may be used. For example, some embodiments use gate valves, globe valves, linear movement valves, among other mechanical valves.

Embodiments including the controller 154 may be used to actively control the valve 152. Accordingly, in such embodiment, the valve 152 may be an active valve, such as an actuatable valve that may be controlled by signals from the controller 154. In some embodiments, the actuatable valve 152 may operate under the control of the controller 154. The valve 152 in this embodiment may comprise, for example, a microelectrical mechanical systems (MEMS) valve, a linear motor valve, a piezoelectric valve, an electromagnetic valve, a pneumatic piston valve, a diaphragm valve, electrical solenoid valve, or other such valve.

The controller 154 may be in communication with the control unit 114 via the transmission module 156. The transmission module 156 may be similar to the transmission module 122. The controller 154 may be constructed as the control unit 114 described above, may be a circuit, a PID controller, or some other type of controller arranged and configured to calculate the IOP from the pressures detected by the sensor or sensors 158. The sensor 158 may be similar to the sensor described above and is arranged to detect the pressure within the eye. In some embodiments, the pressure sensor 158 comprises multiple pressure sensors, including an atmospheric pressure sensor that enables calculation of IOP based off the pressure readings.

Fig. 5 is a graph showing IOP measured over a time period intraoperative or immediately following a phacoemulsification procedure, and identifying a length of time when the IOP matches a target IOP selected to correspond to a natural IOP for a patient. Again, the same principles apply if the system relies upon straight measured pressure. Here, the IOP is controlled by the valve 152 to maintain the IOP at a desired level or within a specific range for a time period so that measurements may be taken with the intra-op diagnostics device 102 (Fig. 1) over a period of time to obtain a more accurate reading of the eye. In one example, the intra-op diagnostics device 102 may be controlled to take a plurality of measurements, which may be averaged by the control unit 114 in order to obtain a more accurate representation of the size of the eye at its natural or normal condition.

Turning now to Fig. 5, at the time T0 along the horizontal axis, the surgeon may pause the surgical procedure or withdraw the irrigation tube from the eye or turn off irrigation flow to the eye. Accordingly, at time T0, the eye is inflated due to the excessively high pressure or IOP maintained during surgery. Also, at time T0, the plug 150 may be inserted into one of the incisions or access ports into the eye.

When the plug 150 includes only a mechanical valve, the valve 152 may be arranged to be open when the eye pressure exceeds a target or desired pressure. The valve 152 may include an indicator, whether visual or audible, that identifies when the valve changes from an open state to a closed state. When the valve changes states indicating that the IOP has reached a target pressure or passes through a target range surrounding a target pressure, the surgeon may control the intra-op diagnostics device 102 to take biometry and/or refractive measurements. In Fig. 5, the target pressure is again identified by IOP X along the IOP axis. When the IOP drops to the target level or range, the mechanical valve 152 closes, slowing the drainage process, and causing the IOP level to more or less plateau. As such, the intraoperative or post-operative biometry and/or refractive measurements can be taken between the times T1 and T2 while the IOP is at a normal state, rather than an elevated state.

When the plug 150 includes the active valve 152, the valve 152 may be open when the eye pressure exceeds a target or desired pressure and may be controlled to close when the pressure reaches a desired or target range. This may include detecting the pressure of the eye with the sensor 158, and based upon the sensed pressure, using the controller 154 to open and close the valve 152. When the valve 152 closes, as may be understood by a visual or audible indicator on the plug 150 itself, the surgeon may operate the intra-op diagnostics device 102 to capture biometry and/or refractive measurements. Since additional drainage may lower the IOP beyond the desired normal pressure, the surgeon may operate the intra-op diagnostics device 102 to capture the measurements within a preset time period after the valve closes. For example, the surgeon may operate to capture with measurements within about 30 seconds or less of the valve 152 closing. Accordingly, again with reference to Fig. 5, as the pressure decreases, the IOP correspondingly decreases. When the pressure or IOP reaches the target pressure, as sensed by the pressure sensor 158, the controller 154 closes the valve 152 to maintain the pressure within the desired range for a period of time. This creates the plateau, and allows the intraoperative or post-operative biometry and/or refractive measurements to be taken over an extended time period time while the IOP is at a normal state, rather than an elevated state. Here, when the period of time is a fixed number, T2 may be determined to be T1+30 seconds. In other embodiments however, T2 may be a point in time more than or less than 30 seconds after T1. In some embodiments, T2 is less than T1+15 seconds. In some embodiments, at the time T2, the plug 150 may provide an addition audible or visual indicator signaling that the measurement capture should cease.

When the plug 150 includes the active valve 152, the controller 154, and the transmission module 156, information relating to the state of the eye or the state of the valve 154 may be transmitted to the intra-op diagnostics device 102. The intra-op diagnostics device 102 may then capture the intraoperative or post-operative measurements at the proper time. This may reduce the need for the surgeon to visually observe the valve 152 to determine its state. Accordingly, again with reference to Fig. 5, the pressure may be measured at the sensor 158. When the pressure sensor 158 detects that pressure decreases to a desired level, the controller 154 operates the valve 152 to close the valve in order to maintain the pressure or IOP at the desired level. At this time, the controller 154 transmits a signal through the transmission module 156 to the intra-op diagnostics device 102 that indicates that the intra-op diagnostics device 102 should capture the biometry and/or refractive measurements of the eye. The signals may indicate that the valve 152 is closed, that pressure is in the desired range, or that the intra-op diagnostics device 102 should take measurements.

With reference to Fig. 5, when the sensor 158 detects that the IOP drops to the target level or range X along the vertical axis, the controller 154, which is in communication with the sensor 156, sends a signal to close the valve 152. It also sends a signal to the transmission module 156 for transmission to the intra-op diagnostics device 102. Accordingly, at time T1, the valve 152 closes and the intra-op diagnostics device 102 may be able to capture biometry and/or refractive measurements. With the valve 152 closed, the pressure may be maintained and the intra-op diagnostics device may take multiple measurements until the time T2. At time T2, the sensor 158 may detect that the pressure is lower than the normal state and the controller 154 may open the valve 152 and/or signal the intra-op diagnostics device 102 to end its measurement taking. Accordingly, intraoperative or post-operative biometry and/or refractive measurements may be obtained when the pressure or IOP is at a normal state, rather than an elevated state. This allows the surgeon to select a lens for implantation that may have a better fit than when the lens is selected based on pre-surgery biometry and/or refractive measurements and a better fit than when the lens is selected based on measurements taken with the eye at an elevated or non-normal pressure or IOP.

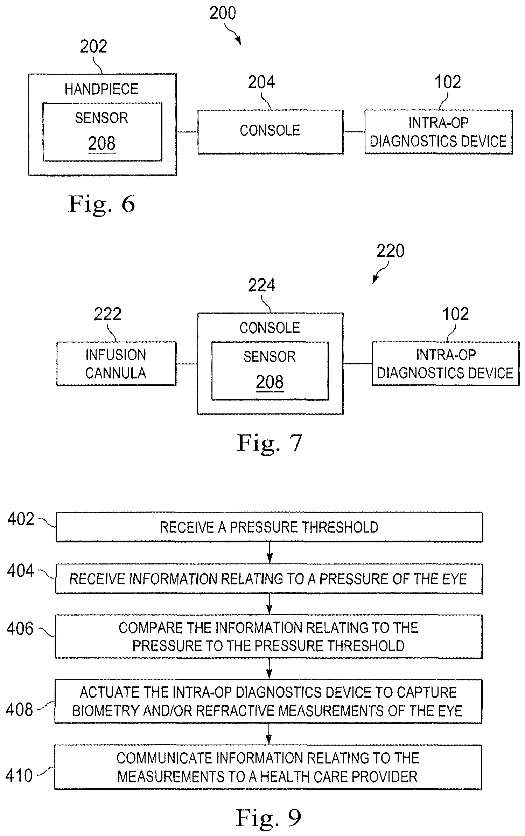

Fig. 6 is a block diagram of a system 200 for intraoperative biometry and/or refractive measurement in cataract surgery and phakic IOL implantation. The system includes a phacoemulsification handpiece 202, a surgical console 204, and the intra-op diagnostics device 102. The phacoemulsification handpiece 202 includes a sensor 208 disposed therein and arranged to measure the pressure within an eye of a phacoemulsification patient. Eye pressure or IOP measurements taken by the handpiece 202 may enable measurements to take place without removing the handpiece 202 from the surgical site and without inserting a separate plug into the surgical site. In addition, data collected at the sensor 208 may be transmitted to the control unit 114 through the surgical console 204 connected to the handpiece 202. Accordingly, in this embodiment, the communication may be carried on wires extending from the handpiece 202 to the surgical console 204. The surgical console 204 may cooperate with the intra-op diagnostics device 102 to capture the measurement data when the pressure and/or IOP is at a desired level.

Fig. 7 is a block diagram of a system 220 for intraoperative biometry and/or refractive measurement in cataract surgery and phakic IOL implantation. The system includes an infusion cannula 222, a surgical console 224, and the intra-op diagnostics device 102. The infusion cannula 222 conveys fluid pressures to a sensor 228 disposed apart from the eye, such as on the surgical console 224. In some embodiments, the infusion cannula 222 has a diameter sized to fit within one of the surgical incisions of the eye. For example, in one embodiment, the infusion cannula 222 fits into the incision having a 1mm diameter. With the infusion cannula 222 in communication with the sensor 228 at the surgical console 224, the control unit 114 on the intra-op diagnostics device 102 may receive data information representing the measured pressure or the IOP of the eye. The surgical console 224 may cooperate with the intra-op diagnostics device 102 to capture the measurement data when the pressure and/or IOP is at a desired level.

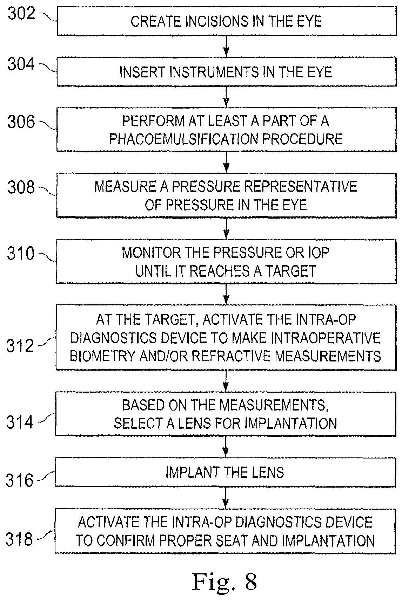

Fig. 8 shows an exemplary method (not part of the invention) of capturing intraoperative biometry and/or refractive measurements in cataract surgery and phakic IOL implantation. Prior to surgery, the surgeon may measure the patient's IOP in order to determine a normal or natural pressure or IOP for the patient. With this information known, the method begins at a step 302. At step 302, the surgeon begins the cataract surgery or phakic IOL implantation surgical procedure by creating an incision in the eye. In some procedures, this may include creating two incisions of different sizes and different purpose. In one aspect, the procedure includes creating a first incision having a length within a range of about 1.8-2.4mm. This incision may be used to access the eye with an instrument, such as a phacoemulsification handpiece, for example. A second incision having a length of about 1mm may be created. This may be used for irrigation tubing or other surgical element.

At a step 304, the surgeon may insert the handpiece with attached irrigation tubing in the eye according to standard surgical procedures. With the instruments in the eye, at a step 306, the surgeon begins emulsifying the lens or the cataracts to prepare the eye for an implantable intraocular lens.

At a step 308, the surgeon may pause the surgical procure to take intraoperative biometry and/or refractive measurements in the eye. In some aspects, this may include removing the tubing from the eye and replacing it with a plug as described herein that may be capable of either passive or active measurement of the eye. As described with reference to the various embodiments herein, the plug may communicate with the control unit 114 of the intra-op diagnostics device 102. In some embodiments, at a step 310, the control unit or other controller may convert the measured pressure to IOP in order to provide a more accurate indication of the state of the eye at a particular time. This may aid when measuring the eye to provide a lens of proper fit. Converting the measured pressure to IOP may include detecting the atmospheric pressure and calculating the IOP based on both the atmospheric pressure and the measured pressure since IOP is a function of both atmospheric and eye pressure. While described as replacing the irrigation tube with the plug, in some aspects, the plug is placed in a separate incision. In such aspects, the fluidics of the phacoemulsification system can be used to deliver a fluid, such as a saline solution to the eye, through an irrigating/aspirating handpiece or infusion cannula inserted in one of incisions in the eye.

Since most surgeries are performed with the eye at an elevated IOP, in order to reduce any chance of collapse during the surgical procedure, it may be necessary to wait while the pressure gradually decreases due to leaking fluid during the paused surgical procedure. As the pressure decreases, the plug may be configured to communicate in real time the detected pressure so that the control unit 114 maintains a real-time indication of the pressure. When IOP is maintained by a phaco surgical console through the infusion cannula and tubing connecting the eye to fluidics system of the surgical console - a surgeon may merely request an appropriate or desired IOP value through a console interface and the console will maintain the IOP at this value.

At a step 310, the control unit 114 may monitor the pressure or IOP until it reaches a pre-established target threshold. The target threshold may be a value or range set to correspond with the normal or natural pressure or IOP for the patient determined in pre-surgical measurements. In other embodiments, the target threshold may be set to correspond with a particular pressure or range that may or may not be unique to the individual's normal pressure. In other embodiments, the control unit 114 does not monitor the target threshold, but the plug provides a visual indication when the target threshold is reached.

At a step 312, when the target pressure is reached, the intra-op diagnostics device 102 may be activated to capture intraoperative biometry and/or refractive measurements. This may be accomplished automatically when the control unit 114 detects that the threshold has been reached, or may accomplished manually when the surgeon receives an indication that the threshold has been reached and then activates the intra-op diagnostics device 102. This indication may be a visual indication, an audible indication, or a tactile indication, for example. The intra-op diagnostics device may take a single measurement or may take multiple measurements over a period of time. In some embodiments, the measurements may be averaged. As described above, in some instances, the measurements may take place for a specific time period. In other embodiments, the pressure may be monitored until the pressure falls below the desired target. In other embodiments, the control unit 114 is programmed to take a designated number of measurements, such as two, three, or four measurements, for example. The example shown in Fig. 1 is based on laser beam ray tracing. In this case, a laser fires a sequence of shots while being scanned over the eye. Each shot produces a spot on the retina, which is being imaged by HD camera or position sensor. The locations of several consecutive shots can be analyzed and used to characterize refraction of the eye. Several methods of refractive characterization of the eye can be used with such pressure valve/sensor disclosed herein. For example, the Shack-Hartmann aberrometer also may be used. This also can be used in combination with an Optical Coherence Tomography device for ocular biometry - to establish IOL location and centration, toric IOL rotation and other characteristics of the eye.

Based on the measurements, the surgeon may select a lens for implantation, at a step 314. The selected lens may have refractive power to best fit the eye refractive properties when the eye is at a normal pressure or IOP. At a step 316, the surgeon may implant the lens in a manner known in the art. This may include removing the plug and restoring the instruments for the surgical procedure. Some embodiments will not require removal of the plug, for example, where the infusion cannula 222 is employed. In some instances, the infusion cannula 222 may be left in place through the surgical procedure. At a step 318, when the implantation process is complete, some surgeons may once again take intraoperative biometry and/or refractive measurements. This may be done to ensure that the lens fits properly and the tissue is disposed as desired after the surgery.

Fig. 9 shows another method (also not part of the invention) for capturing intraoperative biometry and/or refractive measurements. This method includes receiving a pressure threshold at a step 402. The threshold may be received at the control unit 114 from a surgeon during the pre-surgical planning or when programming the intra-op diagnostics device.

At a step 404, the control unit 114 receives information relating to a pressure of an eye during a surgical procedure. Many of the details of the method are described throughout and not all variations are repeated here. As described above, this may be communicated from a plug, the surgical console, the handpiece, or the infusion cannula for example. In addition, these may sent over wire or wireless connection. At a step 406, the control unit 114 compares the information relating to the pressure to the threshold pressure. To do this, the control unit 114 may compare the actual pressure or may compare the IOP, which is a function of the pressure. To obtain the IOP, the system may also measure atmospheric pressure and calculate the IOP based on measured pressure in the eye and based on the measured atmospheric pressure. Based on the comparison, the control unit 114 may control aspects of the system, including, for example, the valve of the plug or other aspects of the plug.

When the control unit 114 determines that the pressure (whether actual or IOP) is below the pressure threshold (whether actual or IOP), the control unit actuates the intra-op diagnostics device to capture biometry and/or refractive measurements of the eye at a step 408. As described above, this may be a single capture or may be a capture in succession over a length of time. In some embodiments, this may continue at regular intervals until the measured pressure falls below the pre-established threshold, which may include falling below a threshold range of pressures. At a step 410, the control unit 114 communicates information relating to the measurements to a health care provider. The health care provider may then make adjustments to the treatment plan, may select a lens sized to fit the eye based on the information, or may take other action.

Although described with the plug being inserted into the incision, other exemplary surgical methods (not part of the invention) include the plug being maintained separately through a separate perforation in the eye globe. In this embodiment, the irrigation tube and the handpiece may be maintained within the eye and the measurements may be captured with those elements within the eye.

The system disclosed herein provide multiple advantages over prior systems as set forth above. In addition, the systems herein include integration of the pressure or IOP sensor/valve with an intra-operative biometry and/or refraction device via wireless communication, improved accuracy of intra-operative biometry through improved accuracy of IOP or pressure target point, decreased dependence on surgical skills due to automation of the procedure, and faster pressure or IOP measurement and adjustment then through manual adjustment and applanation pressure or IOP measurement. In addition, the systems disclosed herein provide actual aqueous pressure measurement inside the eye instead of mechanical resistance of cornea measurement via applanation tonometer.

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A system (100) for capturing intraoperative biometry and/or refractive measurements, comprising:
a pressure sensor (120) associated with the eye and configured to detect a pressure of the eye; and
an intra-op diagnostics device (102) comprising a control unit (114) in communication with the pressure sensor **characterized in that** the control unit is arranged to actuate the intra-op diagnostics device to capture the intraoperative biometry and/or refractive measurements when the sensor detects that the intraocular pressure has decreased from an elevated pressure to a natural intraocular pressure and while the intraocular pressure is maintained at the natural intraocular pressure for a period of time,
a plug (104), the pressure sensor being carried on the plug, and the plug being sized for implantation within the eye of the patient during the surgical procedure,
the plug comprising a valve configured to open and close to increase and decrease drainage from the eye to affect the pressure in the eye.

2. The system of claim 1, wherein the plug (104) comprises a first transmission module (122) in communication with the sensor, and wherein the intra-op diagnostics device (102) comprises a second transmission module (116) in communication with the control unit (114), the first transmission module being in communication with the second transmission module, and the control unit (114) capturing the intraoperative biometry and/or refractive measurements when the communication from the first transmission module indicates that the pressure is below the pressure threshold.

3. The system of claim 1, wherein the valve is configured to close when the pressure in the eye reaches a pressure threshold.

4. The system of claim 3, wherein the pressure threshold is defined in terms of IOP.

## Patentansprüche

1. System (100) zum Erfassen von intraoperativen Biometrie- und/oder refraktiven Messungen, das Folgendes umfasst:
einen Drucksensor (120), der mit dem Auge assoziiert ist und zum Detektieren eines Drucks des Auges ausgebildet ist; und
eine Intra-OP-Diagnosevorrichtung (102), die eine Steuereinheit (114) in Kommunikation mit dem Drucksensor umfasst, **dadurch gekennzeichnet, dass** die Steuereinheit zum Betätigen der Intra-OP-Diagnosevorrichtung zum Erfassen von intraoperativen Biometrie- und/oder refraktiven Messungen eingerichtet ist, wenn der Sensor detektiert, dass der intraokulare Druck von einem erhöhten Druck zu einem natürlichen intraokularen Druck abgenommen hat und, und während der intraokulare Druck für einen Zeitraum auf dem natürlichen intraokularen Druck gehalten wird,
einen Einsatz (104), wobei der Drucksensor auf dem Einsatz getragen wird und der Einsatz zur Implantation innerhalb des Auges des Patienten während der chirurgischen Prozedur getragen wird, wobei der Einsatz ein Ventil umfasst, das zum Öffnen und Schließen ausgebildet ist, um einen Abfluss aus dem Auge zu erhöhen und zu verringern, um den Druck in dem Auge zu beeinflussen.

2. System nach Anspruch 1, wobei der Einsatz (104) ein erstes Übertragungsmodul (122) in Kommunikation mit dem Sensor umfasst und wobei die Intra-OP-Diagnosevorrichtung (102) ein zweites Übertragungsmodul (116) in Kommunikation mit der Steuereinheit (114) umfasst, wobei sich das erste Übertragungsmodul in Kommunikation mit dem zweiten Übertragungsmodul befindet und wobei die Steuereinheit (114) die intraoperativen Biometrie- und/oder refraktiven Messungen erfasst, wenn die Kommunikation von dem ersten Übertragungsmodul angibt, dass der Druck unterhalb des Schwellendrucks liegt.

3. System nach Anspruch 1, wobei das Ventil dazu ausgebildet ist, zu schließen, wenn der Druck in dem Auge eine Druckschwelle erreicht.

4. System nach Anspruch 3, wobei die Druckschwelle hinsichtlich des IOP definiert ist.

## Revendications

1. Système (100) destiné à capturer des mesures biométriques et/ou réfractives peropératoires, comprenant :
un capteur de pression (120) associé à l'œil et configuré pour détecter une pression de l'œil ; et
un dispositif de diagnostic peropératoire (102) comprenant une unité de commande (114) en communication avec le capteur de pression, **caractérisé en ce que** l'unité de commande est agencée pour actionner le dispositif de diagnostic peropératoire pour capturer les mesures biométriques et/ou réfractives peropératoires quand le capteur détecte que la pression intraoculaire a diminué, passant d'une pression élevée à une pression intraoculaire naturelle, et pendant que la pression intraoculaire est maintenue à la pression intraoculaire naturelle pendant un laps de temps ;
un bouchon (104), le capteur de pression étant porté par le bouchon, et le bouchon étant dimensionné pour être implanté à l'intérieur de l'œil du patient pendant la procédure chirurgicale,
le bouchon comprenant une valve configurée pour s'ouvrir et se fermer pour augmenter et diminuer le drainage de l'œil pour affecter la pression dans l'œil.

2. Système de la revendication 1, dans lequel le bouchon (104) comprend un premier module de transmission (122) en communication avec le capteur, et dans lequel le dispositif de diagnostic peropératoire (102) comprend un deuxième module de transmission (116) en communication avec l'unité de commande (114), le premier module de transmission étant en communication avec le deuxième module de transmission, et l'unité de commande (114) capturant les mesures biométriques et/ou réfractives peropératoires quand la communication provenant du premier module de transmission indique que la pression est inférieure au seuil de pression.

3. Système de la revendication 1, dans lequel la valve est configurée pour se fermer quand la pression dans l'œil atteint un seuil de pression.

4. Système de la revendication 3, dans lequel le seuil de pression est défini en terme de pression intraoculaire.
